Europäisches Patentamt

European Patent Office

Office européen des brevets

(19)

(11) Veröffentlichungsnummer : **0 173 149**
**B1**

(12) **EUROPÄISCHE PATENTSCHRIFT**

(45) Veröffentlichungstag der Patentschrift :
20.09.89

(21) Anmeldenummer : 85110050.3

(22) Anmeldetag : 09.08.85

(51) Int. Cl.⁴ : **C 12 N 15/00**, C 12 P 19/34,
C 12 N 1/20, C 12 P 21/00,
C 07 K 15/00 // C12R1:19

(54) Synthetische Regulationsregion.

(30) Priorität : 21.08.84 DE 3430683

(43) Veröffentlichungstag der Anmeldung :
05.03.86 Patentblatt 86/10

(45) Bekanntmachung des Hinweises auf die Patenterteilung : 20.09.89 Patentblatt 89/38

(84) Benannte Vertragsstaaten :
AT BE CH DE FR GB IT LI LU NL SE

(56) Entgegenhaltungen :
EP-A- 0 067 540
DE-A- 3 336 586

(73) Patentinhaber : HOECHST AKTIENGESELLSCHAFT
Postfach 80 03 20
D-6230 Frankfurt am Main 80 (DE)

(72) Erfinder : Engels, Joachim, Prof. Dr.
Feldbergstrasse 1
D-6242 Kronberg (Taunus) (DE)
Erfinder : Uhlmann, Eugen, Dr.
Washington Street 287
Belmont, Mass. 02178 (US)
Erfinder : Leineweber, Michael, Dr.
Heimchenweg 74
D-6230 Frankfurt am Main 80 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der gentechnischen Herstellung eukaryotischer Polypeptide in Bakterien, insbesondere E. coli, wird das « heterologe » Gen, welches für das gewünschte eukaryotische Polypeptid codiert, in einen geeigneten Vektor eingebaut und dieser Hybridvektor dann in den bakteriellen Wirt eingebracht. Es müssen jedoch eine Reihe von Voraussetzungen vorliegen, damit dieses heterologe Gen die Produktion des gewünschten Polypeptids bewirken kann. Eine wesentliche Voraussetzung ist eine funktionierende Regulationsregion, bestehend aus einem Operator, einem Promotor und einer sogenannten « Shine-Dalgarno-Sequenz », auch SD-Sequenz oder, vereinfachend (da an das Ribosom erst die entsprechende Sequenz der mRNA bindet) im folgenden « ribosomale Bindungsstelle » genannt.

Die richtige Expression des Gens, also die Produktion des gewünschten Polypeptids, setzt ein Erkennen des Promotors durch den bakteriellen Wirt voraus. Das wirtseigene Enzym RNA-Polymerase erkennt eine Teilsequenz in der DNA des Promotors und bindet an diese Teilsequenz. Hierdurch wird eine Öffnung der doppelsträngigen DNA in dieser Region bewirkt, worauf die Synthese der mRNA am codierenden Strang (Transkriptionsstrang) beginnt.

Der Operator, der häufig mit dem Promotor überlappt, wird durch ein wirtseigenes Repressor-Protein erkannt. Durch die mehr oder weniger wirksame Bindung dieses Repressor-Proteins an den Operator wird die Transkriptionshäufigkeit reguliert. Dieses System wird durch Induktoren (Inducer-Moleküle) beeinflußt, die das Repressor-Protein binden und so den Operator aktivieren.

Die ribosomale Bindungsstelle ist schließlich dafür verantwortlich, daß der zunächst synthetisierte Teil der mRNA eine RNA-Sequenz für die Bindung an das Ribosom erhält, an dem die Translation, also die « Übersetzung » in das Polypeptid, stattfindet.

Die Regulationsregion ist somit zuständig für die Expression des Gens in das gewünschte Polypeptid über die Stufe der Transkription (Umschreibung der DNA in mRNA) und die anschließende Translation. Wichtig für den Aufbau einer solchen Regulationsregion ist außer der Folge der Nucleotide die Geometrie, also die räumliche Zuordnung von Promotor, Operator und ribosomaler Bindungsstelle.

Im folgenden bezieht sich die Numerierung der Nukleotide auf die Stelle des Transkriptionsstartes (Null), wobei wie üblich von 5'- in 3'-Richtung gezählt wird.

Natürliche Promotoren für die E. coli-RNA-Polymerase zeigen zwei Regionen konservierter DNA-Sequenzen. Es ist dies zum einen die -35-Region und andererseits die -10-Region, auch « Pribnow-Schaller-Box » genannt, wobei sich die Bezifferung auf die genannte Nucleotid-Numerierung bezieht, d. h. daß diese Regionen vor dem Transkriptionsstart angeordnet sind.

Die erfindungsgemäße Regulationssequenz stellt eine Modifikation der natürlichen Regulationssequenzen dar, die eine optimale Bindung der RNA-Polymerase an den Promotor und eine effektive Nutzung des Operators gewährleistet. Die erfindungsgemäße Regulationssequenz kann entweder direkt vor das heterologe Gen gesetzt werden, worauf das gewünschte Polypeptid (mit Methionin am Aminoterminus) exprimiert wird, oder es wird ein Bakteriengen — ganz oder teilweise — vor das heterologe Gen geschaltet, wodurch ein Fusionsprotein exprimiert wird, wobei ein Bakterienprotein-Anteil an den Aminoterminus des gewünschten Polypeptids gebunden ist.

Die erfindungsgemäße synthetische Regulationsregion für die Expression heterologer Gene in E. coli, enthaltend einen Promotor, einen modifizierten lac-Operator und eine ribosomale Bindungsstelle, ist gekennzeichnet durch die folgenden Merkmale :

a) die -35-Region im Promotor hat die Nucleotid-Sequenz (codierender Strang)

TTGACAT oder CTTGACAT

b) die -10-Region im Promotor hat die Nucleotid-Sequenz (codierender Strang)

GTATAAT,

c) zwischen der -35- und der -10-Region ist eine Spacer-Gruppierung von 14 bis 17 Basenpaaren und
d) zwischen der ribosomalen Bindungsstelle und dem ATG-Startcoden ist eine Spacer-Gruppierung von 6 bis 14 Basenparen angeordnet.

Weitere Ausgestaltungen dieser Erfindung sind in den Patentansprüchen niedergelegt.

Neben den bereits genannten Vorteilen zeichnet sich die erfindungsgemäße Regulationsregion dadurch aus, daß sie sehr variabel ist und aufgrund einer Reihe singulärer Restriktionsschnittstellen die einzelnen Elemente, nämlich Promotor, Operator und ribosomale Bindungsstelle, sich herausschneiden und mit bekannten Systemen kombinieren lassen. Weiterhin kann durch Modifikation der Spacer-Gruppierungen die Geometrie variiert und an die Gegebenheiten des Einzelfalles noch besser angepaßt werden.

Die Konstruktion der erfindungsgemäßen Regulationsregion erfolgt vorzugsweise vollsynthetisch. Hierfür können die bekannten DNA-Synthesemethoden eingesetzt werden, beispielsweise die Phosphitmethode.

Die DNA-Sequenz I (Anhang) zeigt eine vorteilhafte Ausgestaltung der gesamten Regulationsregion. Die DNA-Sequenzen II a bis II h zeigen spezifische, bevorzugte Ausgestaltungen der Sequenz I. Zur Synthese der Sequenzen I bzw. II werden am 5'- und 3'-Ende jeweils noch einige Nucleotidpaare angefügt, die den Angriff von Restriktionsenzymen zum « Zurechtschneiden » erlauben. Die DNA-Sequenz IIIa ist zusätzlich vollständig aufgeführt, wobei am 5'- und 3'-Ende je drei Nucleotidpaare angesetzt sind. Selbstverständlich könnten hier auch andere bzw. mehrere Nucleotidpaare angefügt werden.

In den folgenden Beispielen werden spezielle Ausgestaltungen der Erfindung im einzelnen erläutert, woraus sich die Vielzahl der möglichen Abwandlungen und Kombinationen für den Fachmann ergibt. Prozentangaben beziehen sich hierbei auf das Gewicht, wenn nichts anderes angegeben ist.

Beispiel 1

Synthese der DNA-Sequenz IIa

a) Chemische Synthese eines einzelsträngigen Oligonucleotids

Am Beispiel des Genbausteins Ia, der die Nucleotide 1-19 (und zusätzlich drei weitere am 5'-Ende zur Ermöglichung des Angriffs von Bam HI) des codierenden Strangs umfaßt, wird die Synthese der Genbausteine erläutert. Nach bekannten Methoden (M.J. Gait et al., Nucleic Acids Res. 8 (1980) 1081-1096) wird das am 3'-Ende stehende Nucleosid, im vorliegenden Falle also Thymidin (Nucleotid Nr. 19), an Kielselgel ($®$FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Thymidin wird in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylaminogruppe acyliert.

In den folgenden Syntheseschritten wird die Basenkomponente als 5'-O-Dimethoxytrityl-nucleosid-3'-phosphorigsäuremonomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

100 mg des polymeren Trägers, der 4 µmol Thymidin gebunden enthält, werden nacheinander mit den folgenden Agentien behandelt :

a) Nitromethan
b) gesättigte Zinkbromidlösung in Nitromethan mit 1 % Wasser
c) Methanol
d) Tetrahydrofuran
e) Acetonitril
f) 80 µmol des entsprechenden Nucleosidphosphits und 400 µmol Tetrazol in 1 ml wasserfreiem Acetonitril (5 Minuten)
g) 20 % Acetanhydrid in Tetrahydrofuran mit 40 % Lutidin und 10 % Dimethylaminopyridin (2 Minuten)
h) Tetrahydrofuran
i) Tetrahydrofuran mit 20 % Wasser und 40 % Lutidin
j) 3 % Jod in Kollidin/Wasser/Tetrahydrofuran im Volumenverhältnis 5 : 4 : 1
k) Tetrahydrofuran und
l) Methanol

Unter « Phosphit » wird hierbgei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion (b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten. Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib-Ih synthetisiert, deren Nucleotidfolge aus der DNA-Sequenz IIa hervorgeht.

b) Enzymatische Verknüpfung der einzelsträngigen Oligonucleotide

Zur Phosphorylierung der Oligonucleotide am 5'-Terminus werden je 1,0 nmol der Oligonucleotide Ib und Ic unter Zusatz von 10 nmol Adenosintriphosphat mit 6 Einheiten T4-Polynucleotidkinase in 20 µl 50 mM Tris-HCl-Puffer (pH 7,6), 10 mM Magnesiumchlorid und 10 mM Dithiothreitol (DTT) 30 Minuten bei 37 °C behandelt (C.C. Richardson, Progress in Nucl. Acid Res. 2 (1972) 825). Das Enzym wird durch fünfminütiges Erhitzen auf 95 °C desaktiviert.

Analog werden die Oligonucleotide If und Ig phosphoryliert.

Die Oligonucleotide Ia und Id und die phosphorylierten Oligonucleotide Ib und Ic werden in 40 µl 50 mM Tris-HCl-Puffer 5 Minuten auf 95 °C erhitzt, worauf man sie langsam auf Raumtemperatur abkühlen läßt. Hierzu fügt man 20 mM Magnesiumchlorid, 10 mM DTT und 1 mM ATP und läßt mit 100 Einheiten T4-DNA-Ligase bei 25 °C 16 Stunden lang reagieren.

Analog werden die Fragmente Ie und Ih mit den phosphorylierten Fragmenten If und Ig verknüpft.

Das Produkt der Ligasereaktion der Oligonucleotide Ia bis Id (Genfragment A) wird gefriergetrocknet und in 100 µl einer Pufferlösung (150 mM NaCl, 10 mM Tris-HCl, pH 7,6, 6 mM Magnesiumchlorid), die 200 Einheiten der Endonuclease Bam HI enthält, bei 37 °C 3 Stunden inkubiert.

Das Produkt der Ligasereaktion der Oligonucleotide Ie bis Ih (Genfragment B) wird gefriergetrocknet und in 100 µl einer Pufferlösung (100 mM Tris-HCl, pH 7,5, 50 mM NaCl, 5 mM Magnesiumchlorid), die 200 Einheiten der Endonuclease Eco RI enthält, bei 37 °C 3 Stunden inkubiert. Nach dem Stoppen der Enzymverdauung dur 2 minütiges Erhitzen auf 95 °C werden die geschnittenen Genfragmente A und B durch Gelelektrophorese auf 15 %igem Polyacrylamidgel (ohne Harnstoffzusatz, 20 × 40 cm, 2 mm Dicke), gereinigt, wobei als Markersubstanz pBR 322 (Fa. Biolabs), geschnitten mit Hae III, dient. Nach Extraktion der DNA-Banden und Reinigung über ®Sephadex G50 und Sep Pak (Fa. Waters) werden die Genfragmente A und B durch « blunt end ligation » verknüpft. Man erhält so eine synthetische Regulationsregion entsprechend der DNA-Sequenz IIa, die am 5'-Ende des codierenden Strangs eine Verlängerung für den Angriff von Baum HI und am 3'-Ende des codierenden Strangs eine Verlängerung für den Angriff von Eco RI aufweist.

## Beispiel 2

Hybridplasmide, die die synthetische Kontrollregion enthalten

### a) Einbau der Kontrollregion in pUC 8

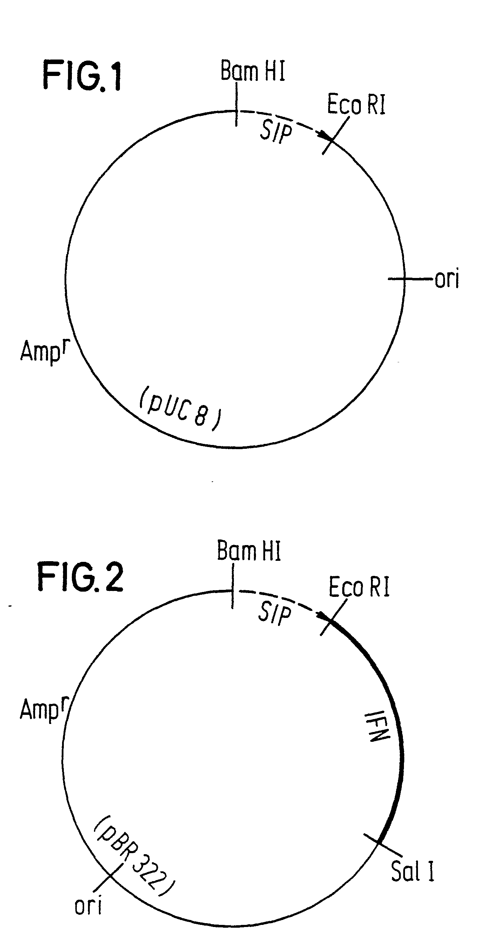

Das handelsübliche Plasmid pUC 8 wird in bekannter Weise mit den Restriktionsendonucleasen Eco RI und Bam HI geöffnet und über 1 %ige niedrigschmelzende Agarose-Gele von den herausgeschnittenen Oligonucleotiden abgetrennt. Die Wiedergewinnung des geschnittenen Plasmids erfolgt nach Auflösen des Gels bei erhöhter Temperatur nach Maßgabe der Hersteller. 1 µg des so geöffneten pUC 8-Plasmids werden mit 10 µg der synthetischen Kontrollregion mittels T4 DNA-Ligase bei 14 °C über Nacht ligiert. Man erhält so ein modifiziertes pUC 8-Plasmid mit der integrierten Kontrollregion. Dieses Hybridplasmid ist in der Figur 1 dargestellt, in der die Kontrollregion als SIP bezeichnet ist, was für « synthetischer idealisierter Promotor » steht.

### b) Transformation

Zellen des Stammes E. coli K 12 werden durch Behandeln mit einer 70 mM Calciumchloridlösung kompetent gemacht und mit der Suspension des Hybridplasmides in 10 mM Tris-HCl-Puffer (pH 7,5) der 70 mM an Calciumchlorid ist, versetzt. Die transformierten zellen werden auf Ampicillinresistenz selektiert und die insertierte Sequenz durch Sequenzierung nach Maxam-Gilbert verifiziert (A. Maxam und W. Gilbert, Proc. Natl. Acad. Sci. USA 74, 560-564 (1977).

## Beispiel 3

Expressionsplasmide, die die synthetische Kontrollregion enthalten

Das handelsübliche Plasmid pBR 322 wird mit den Restriktionsenzymen Bam HI und Sal I geöffnet und wie oben beschrieben über ein 1 %iges Agarose-Gel gereinigt. Die synthetische Kontrollregion wird aus dem modifizierten pUC 8-Derivat durch Schneiden mit den Enzymen Eco RI und Bam HI reisoliert, auf 10 %igen Polyacrylamidgelen gereinigt und durch anschließende Elektroelution wiedergewonnen. In analoger Weise wird das γ-Interferongen aus entsprechenden Hybridplasmiden durch Schneiden mit den Restriktionsenzymen Eco RI und Sal I und Reinigen über ein 2 %iges Gel aus niedrigschmelzender Agarose isoliert. Als Hybridplasmid, das das γ-Interferongen enthält, wird das Plasmid pMX 2 eingesetzt, dessen Herstellung in der DE-A-3 409 966.2 beschrieben ist. Es kann jedoch auch das in der EP-A-0 095 350 beschriebene Plasmid eingesetzt werden.

Das linearisierte Plasmid pBR 322, die synthetische Kontrollregionen und das γ-Interferongen werden dann in bekannter Weise ligiert, wobei das Plasmid gemäß Figur 2 erhalten wird.

Beispiel 4

Aktivitätsvergleich der synthetischen Kontrollregion mit der bekannten starken Kontrollregion tac, beschrieben u. a. in der EP-A-67 540.

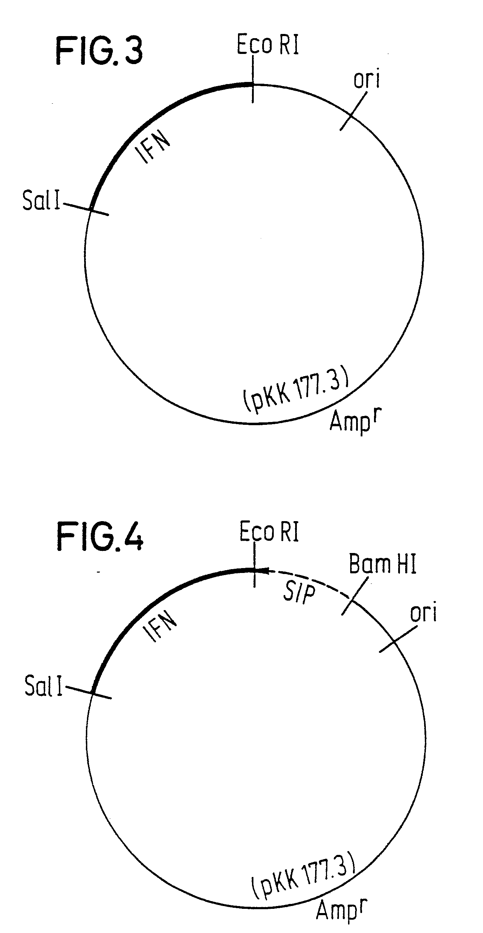

Das literaturbekannte Plasmid pKK 177.3 (E. Amann et al., Gene 25, 167 (1983) wird mit den Restriktionsenzymen Eco RI und Sal I wie oben beschrieben geschnitten und das γ-Interferongen eingebaut, wodurch dieses an die tac-Kontrollregion gekopplet wird. Man erhält das Plasmid gemäß Figur 3.

Zur Entfernung der tac-Kontrollregion aus pKK 177.3 wird dieses mit Bam HI und Sal I verdaut. Das so linearisierte Plasmid wird wie oben beschrieben mit dem γ-Interferongen und der synthetischen Kontrollregion (siehe DNA Sequenz IIa) ligiert. Man erhält das Hybridplasmid gemäß Figur 4.

Die Hybridplasmide gemäß Figur 3 und Figur 4 werden in E. coli K 12 transformiert und die Bakterien in 2 YT-Medium (Miller, Experiments in Molecular Genetic ; 1972) angezogen, bis eine optische Dichte von 1 bei 578 nm in der Schüttelkultur erreicht ist. Zu einem Teil der Bakterienkultur wird 0,1 mM IPTG (Isopropyl-β-thiogalaktopyranosid) zugesetzt und hierdurch die Synthese von γ-Interferon 2 Stunden lang induziert.

Anschließend werden die Bakterien abzentrifugiert und durch Behandlung mit Lysozym, EDTA und Ultraschall aufgeschlossen (Maniatis et al., Molecular Cloning, Cold Spring Harbor, 1982). Die Interferontiter der Bakterienlysate werden mit einem käuflichen Radioimmunoassay (Celltech) wie folgt ermittelt :

Plasmid gemäß Figur 3 : $1 \times 10^5$ Einheiten pro ml
Plasmid gemäß Figur 4 : $1,5 \times 10^5$ Einheiten pro ml.

Im Vergleich zu der bekanntermaßen exzellenten tac-Region wird somit mit der erfindungsgemäßen Kontrollregion eine um 50 % höhere γ-Interferon-Ausbeute erhalten.

(Siehe Formeln Seite 6 ff.)

DNA-Sequenz I (codierender Strang) :

5' GGATCCTAAATAAATTCTTGACATTT1TTAA2TAATTTGGTATAATGT3T

4GAATTG5GAGCG6T7ACAATT8C9C10G11G12T13TA14TT15 (ATG) 3'

| | | |
|---|---|---|
| 1 = T oder G | 7 = A oder C | 11 = AG oder GA |
| 2 = A oder C | 8 = T oder direkte Bindung | 12 = A oder G |
| 3 = G oder C | 9 = A oder TAGA | 13 = C oder T |
| 4 = G oder A | 10 = A, TTTAAA, AAGCTT | 14 = GAA oder AGC |
| 5 = T oder C |     oder AAGCTA | 15 = C oder direkte Bindung |
| 6 = C, GA oder GAA | | |

DNA-Sequenzen IIa-h :

| | 1 | 2 | 5 | 6 | 7 | 8 | 10 | 11 | 12 | 13 | 14 | 15 | |
|---|---|---|---|---|---|---|---|---|---|---|---|---|---|
| IIa | T | A | T | GAA | A | T | A | AG | A | C | GAA | C | 3 = G |
| b | T | A | T | GAA | A | T | TTTAAA | AG | A | C | GAA | C | 4 = G |
| c | G | C | T | GAA | A | T | TTTAAA | AG | A | C | GAA | C | 9 = A |
| d | G | C | T | GAA | A | – | AAGCTT | AG | A | C | GAA | C | |
| e | G | C | C | GAA | A | – | AAGCTT | AG | A | C | GAA | C | |
| f | G | C | T | C | C | – | AAGCTT | AG | A | C | GAA | C | |
| g | G | C | T | GA | C | – | AAGCTT | AG | A | C | GAA | C | |
| h | G | C | T | GA | C | – | AAGCTA | GA | G | T | AGC | – | |

EP 0 173 149 B1

## EP 0 173 149 B1

### DNA-Sequenz IIa

```
                Bam HI
         ◄───────────────────── Ia, ──────────────────────►    ◄─
           1                  10                  20
       5' A G C G G A T C C T A A A T A A A T T C T T G A C A T
       3' T C G C C T A G G A T T T A T T T A A G A A C T G T A
         ─────────────────────── Ib, ──────────────────────
              Aha III
         ─────────────────────── Ic, ──────────────────────
              30                  40
       5' T T T T A A A T A A T T G G T A T A A T G T
       3' A A A A T T T A T T A A A C C A T A T T A C A
         ──── Ib, ───►  ◄─────────────── Id, ──────────────
         ───►  ◄───────────────── Ie, ─────────────────►  ◄─ Ig, ──
              50                  60                  70
       5' G T G G A A T T G T G A G C G G A A T A A C A A T T
       3' C A C C T T A A C A C T C G C C T T A T T C T T A A
         ───►  ◄──────────────── If, ───────────────────
                                      Eco RI
         ─────────────────── Ig, ───────────────────────►
              80                  90
       5' C A C A G A G G A T C T A G A A T T C A C T
       5' G T G T C T C C T A G A T C T T A A G T G A
         ───►  ◄───────────── Ih, ──────────────────────►
```

**Patentansprüche** (für die Vertragsstaaten : BE, CH, DE, FR, GB, IT, LI, LU, NL und SE)

1. Synthetische Regulationsregion für die Expression heterologer Gene in E. coli, enthaltend einen Promotor, einen modifizierten lac-Operator und eine ribosomale Bindungsstelle, dadurch gekennzeichnet, daß

    a) die -35-Region im Promotor die Nucleotid-Sequenz (codierender Strang)

<div align="center">TTGACAT oder CTTGACAT</div>

hat,

    b) die -10-Region im Promotor die Nucleotid-Sequenz (codierender Strang)

<div align="center">GTATAAT</div>

hat,

    c) zwischen der -35- und -10-Region eine Spacer-Gruppierung von 14 bis 17 Basenpaaren ist und

    d) zwischen der ribosomalen Bindungsstelle und dem ATG-Startcodon eine Spacer-Gruppierung von 6 bis 14 Basenpaaren angeordnet ist.

2. Regulationsregion nach Anspruch 1, dadurch gekennzeichnet, daß die Spacer-Gruppierung zwischen der -35 und der -10-Region 15 Basenpaare aufweist und/oder A,T-reich ist.

3. Regulationsregion nach Anspruch 1 oder 2, dadurch gekennzeichnet, daß die Spacer-Gruppierung zwischen der ribosomalen Bindungsstelle und dem Startcodon 10 Basenpaare aufweist und/oder A,T-reich ist.

<div align="center">7</div>

4. Regulationsregion nach einem oder mehreren der vorhergehenden Ansprüche, dadurch gekennzeichnet, daß die ribosomale Bindungsstelle purinreich ist.

5. Regulationsregion nach einem oder mehreren der vorhergehenden Ansprüche, dadurch gekennzeichnet, daß der modifizierte lac-Operator die DNA-Sequenz I oder IIa (Anhang) aufweist.

6. Genstruktur, enthaltend eine Regulationsregion nach Anspruch 1 bis 5,

7. Hybridvector, enthaltend eine Genstruktur nach Anspruch 6.

8. E. coli, enthaltend einen Hybridvector nach Anspruch 7.

9. Verfahren zur Herstellung eines Polypeptids, dadurch gekennzeichnet, daß ein E. coli-Stamm nach Anspruch 8 eingesetzt wird.


**Patentansprüche** (für den Vertragsstaat AT)

1. Verfahren zur Expression heterologer Gene in E. coli, dadurch gekennzeichnet, daß man in Ableserichtung vor das heterologe Gen eine synthetische Regulationsregion setzt, die einen Promotor, einen modifizierten lac-Operator und eine ribosomale Bindungsstelle enthält, in welcher

a) die -35-Region im Promotor die Nucleotid-Sequenz (codierender Strang)

TTGACAT oder CTTGACAT

hat,

b) die -10-Region im Promotor die Nucleotid-Sequenz (codierender Strang)

GTATAAT

hat,

c) zwischen der -35- und -10-Region eine Spacer-Gruppierung von 14 bis 17 Basenpaaren ist und

d) zwischen der ribosomalen Bindungsstelle und dem ATG-Startcodon eine Spacer-Gruppierung von 6 bis 14 Basenpaaren angeordnet ist.

2. Verfahren nach Anspruch 1, dadurch gekennzeichnet, daß die Spacer-Gruppierung zwischen der -35 und der -10-Region 15 Basenpaare aufweist.

3. Verfahren nach Anspruch 1 oder 2, dadurch gekennzeichnet, daß die Spacer-Gruppierung zwischen der -35 und der -10-Region A,T-reich ist.

4. Verfahren nach einem oder mehreren der vorhergehenden Ansprüche, dadurch gekennzeichnet, daß die Spacer-Gruppierung zwischen der ribosomalen Bindungsstelle und dem Startcodon 10 Basenpaare aufweist.

5. Verfahren nach einem oder mehreren der vorhergehenden Ansprüche, dadurch gekennzeichnet, daß die Spacer-Gruppierung zwischen der ribosomalen Bindungsstelle und dem Startcodon A,T-reich ist.

6. Verfahren nach einem oder mehreren der vorhergehenden Ansprüche, dadurch gekennzeichnet, daß die ribosomale Bindungsstelle purinreich ist.

7. Verfahren nach einem oder mehreren der vorhergehenden Ansprüche, dadurch gekennzeichnet, daß dieser modifizierte lac-Operator die DNA-Sequenz I oder IIa (Anhang) aufweist.


**Claims** (for the Contracting States : BE, CH, DE, FR, GB, IT, LI, LU, NL and SE)

1. A synthetic regulation region for the expression of heterologous genes in E. coli, containing a promotor, a modified lac operator and a ribosome-binding site, characterized in that

a) the -35 region in the promotor has the nucleotide sequence (coding strand)

TTGACAT or CTTGACAT

b) the -10 region in the promotor has the nucleotide sequence (coding strand)

GTATAAT,

c) a spacer group of 14 to 17 base-pairs is between the -35 and the -10 regions, and

d) a spacer group of 6 to 14 base-pairs is located between the ribosome-binding site and the ATG start codon.

2. A regulation region according to claim 1, characterized in that the spacer group between the -35 and the -10 regions has 15 base-pairs and/or is rich in A and T.

3. A regulation region according to claim 1 or 2, characterized in that the spacer group between the ribosome-binding site and the start codon has 10 base-pairs and/or is rich in A and T.

4. A regulation region according to one or more of the preceding claims, characterized in that the ribosome-binding site is rich in purines.

8

EP 0 173 149 B1

5. A regulation region according to one or more of the preceding claims, characterized in that the modified lac operator has the DNA sequence I or IIa (Appendix).

6. A gene structure containing a regulation region according to claim 1 to 5.

7. A hybrid vector containing a gene structure according to claim 6.

8. E. coli containing a hybrid vector according to claim 7.

9. A process for the preparation of a polypeptide, characterized in that an E. coli strain according to claim 8 is used.

**Claims** (for the Contracting State AT)

1. A process for the expression of heterologous genes in E. coli, characterized by placing, upstream of the heterologous gene in the direction of reading, a synthetic regulation region which contains a promotor, a modified lac operator and a ribosome-binding site, in which

a) the -35 region in the promotor has the nucleotide sequence (coding strand)

TTGACAT or CTTGACAT

b) the -10 region in the promotor has the nucleotide sequence (coding strand)

GTATAAT,

c) a spacer group of 14 to 17 base-pairs is between the -35 and the -10 regions, and

d) a spacer group of 6 to 14 base-pairs is located between the ribosome-binding site and the ATG start codon.

2. The process according to claim 1, characterized in that the spacer group between the -35 and the -10 regions has 15 base-pairs.

3. The process according to claim 1 or 2, characterized in that the spacer group between -35 and the -10 regions is rich in A and T.

4. The process according to one or more of the preceding claims, characterized in that the spacer group between the ribosome-binding site and the start codon has 10 base-pairs.

5. The process according to one or more of the preceding claims, characterized in that the spacer group between the ribosome-binding site and the start codon is rich in A and T.

6. The process according to one or more of the preceding claims, characterized in that the ribosome-binding site is rich in purines.

7. The process according to one or more of the preceding claims, characterized in that this modified lac operator has the DNA sequence I or IIa (Appendix).

**Revendications** (pour les Etats contractants : BE, CH, DE, FR, GB, IT, LI, LU, NL, SE)

1. Région synthétique de régulation pour l'expression de gènes hétérologues dans E. coli, contenant un promoteur, un opérateur lac modifié et un site de liaison ribosomique, caractérisée en ce que

a) la région -35 dans le promoteur comporte la séquence de nucléotides (brin codant)

TTGACAT ou CTTGACAT,

b) la région -10 dans le promoteur comporte la séquence de nucléotides (brin codant)

GTATAAT,

c) entre la région -35 et la région -10, se trouve un groupement espaceur de 14 à 17 paires de bases, et

d) entre le site de liaison ribosomique et le codon d'initiation ATG est disposé un groupement espaceur de 6 à 14 paires de bases.

2. Région de régulation selon la revendication 1, caractérisée en ce que le groupement espaceur entre la région -35 et la région -10 comporte 15 paires de bases et/ou est riche en A, T.

3. Région de régulation selon la revendication 1 ou 2, caractérisée en ce que le groupement espaceur entre le site de liaison ribosomique et le codon d'initiation comporte 10 paires de bases et/ou est riche en A, T.

4. Région de régulation selon une ou plusieurs des revendications précédentes, caractérisée en ce que le site de liaison ribosomique est riche en bases puriques.

5. Région de régulation selon une ou plusieurs des revendications précédentes, caractérisée en ce que l'opérateur lac modifié comporte la séquence d'ADN I ou IIa (voir appendice).

6. Structure génique contenant une région de régulation selon l'une des revendications 1 à 5.

9

7. Vecteur hybride contenant une structure génique selon la revendication 6.

8. E. coli contenant un vecteur hybride selon la revendication 7.

9. Procédé pour la préparation d'un polypeptide, caractérisé en ce que l'on utilise une souche de E. coli selon la revendication 8.


**Revendications** (pour l'Etat contractant AT)

1. Procédé pour l'expression de gènes hétérologues dans E. coli, caractérisé en ce que, dans le sens de lecture, on introduit en amont du gène hétérologue une région synthétique de régulation qui contient un promoteur, un opérateur lac modifié et un site de liaison ribosomique, dans laquelle

a) la région -35 dans le promoteur comporte la séquence de nucléotides (brin codant)

TTGACAT ou CTTGACAT

b) la région -10 dans le promoteur comporte la séquence de nucléotides (brin codant)

GTATAAT,

c) entre la région -35 et la région -10, se trouve un groupement espaceur de 14 à 17 paires de bases, et

d) entre le site de liaison ribosomique et le codon d'initiation ATG est disposé un groupement espaceur de 6 à 14 paires de bases.

2. Procédé selon la revendication 1, caractérisé en ce que le groupement espaceur entre la région -35 et la région -10 comporte 15 paires de bases.

3. Procédé selon la revendication 1 ou 2, caractérisé en ce que le groupement espaceur entre la région -35 et la région -10 est riche en A, T.

4. Procédé selon une ou plusieurs des revendications précédentes, caractérisé en ce que le groupement espaceur entre le site de liaison ribosomique et le codon d'initiation comporte 10 paires de bases.

5. Procédé selon une ou plusieurs des revendications précédentes, caractérisé en ce que le groupement espaceur entre le site de liaison ribosomique et le codon d'initiation est riche en A, T.

6. Procédé selon une ou plusieurs des revendications précédentes, caractérisé en ce que le site de liaison ribosomique est riche en bases puriques.

7. Procédé selon une ou plusieurs des revendications précédentes, caractérisé en ce que cet opérateur lac modifié comporte la séquence d'ADN I ou IIa (voir appendice).

FIG.1

FIG.2

FIG. 3

FIG. 4